# EUROPEAN PATENT SPECIFICATION

(11) **EP 3 549 564 B1**
(45) Date of publication and mention of the grant of the patent: **04.11.2020**
(21) Application number: 16925832.4
(22) Date of filing: 28.12.2016
(51) Int. Cl.: A61F 13/49, A61F 13/56, A61F 13/00

(54) **ABSORBENT ARTICLE**
ABSORBIERENDER ARTIKEL
ARTICLE ABSORBANT

(43) Date of publication of application: 09.10.2019
(73) Proprietor: Unicharm Corporation, Ehime 799-0111 (JP)
(72) Inventor: SAKAGUCHI, Satoru, Kanonji-shi Kagawa 769-1602 (JP); TAKAHASHI, Maika, Kanonji-shi Kagawa 769-1602 (JP)
(74) Representative: Dolleymores
(86) International application number: PCT/JP2016/089178
(87) International publication number: WO 2018/123047

(56) References cited:
- JP-A- 2005 046 225
- JP-A- 2014 064 627
- US-A1- 2004 073 188
- US-A1- 2005 027 279
- US-A1- 2005 043 699
- US-A1- 2014 330 236

## Description

### FIELD

The present invention relates to an absorbent article.

### BACKGROUND

Absorbent articles such as disposable diapers are known. Such absorbent articles include, for example, an absorbent body and a pair of side flaps extending outward from both ends in the widthwise direction of the absorbent body. Patent Literature 1 discloses a disposable diaper (absorbent article) as one such absorbent article. In Patent Literature 1, each side flap is stretchable in the widthwise direction due to a stretching member. In the thickness direction, each side flap (flap section) is situated on the non-skin side of an absorber. In the lengthwise direction, the end on the dorsal side of the absorber is situated further toward the abdominal side than belt-shaped regions where the engaging members of the side flaps in the widthwise direction are to be connected together.

### CITATION LIST

### PATENT LITERATURE

PTL 1: Japanese Unexamined Patent Publication No. 2016-21981

US 2005/0043699 A1, US 2004/0073188 A1 and US 2005/0027279 A1 each disclose a disposable diaper comprising a pair of stretchable wings extending outward from transversely opposite side edge portions of a rear waist region in the transverse direction.

### SUMMARY

### TECHNICAL PROBLEM

In the side flaps of the disposable diaper described in Patent Literature 1, the stretching member is only disposed in approximately the center region, in the lengthwise direction, whereas both outer sides include only nonwoven fabrics. When such a disposable diaper is fitted onto a child, the stretching member is pulled outward in the widthwise direction via the engaging members (fastening tape), causing the absorbent body to be pulled to both outer sides in the widthwise direction. In the absorbent body, the dorsal side end in the lengthwise direction which is further toward the dorsal side than the portions pulled by the stretching member is not directly pulled by the stretching member, but instead it is pulled in the direction of the stretching member via the nonwoven fabric of each side flap. At the dorsal side end in the lengthwise direction of the absorbent body, therefore, both ends in the widthwise direction are not only pulled outward in the widthwise direction but are also pulled to the abdominal side in the lengthwise direction. When this occurs, the dorsal side end in the lengthwise direction of the absorbent body either collapses to the non-skin side or collapses to the skin side, and the disposable diaper described in Patent Literature 1 tends to easily collapse to the non-skin side when it is pulled outward in the widthwise direction. When the dorsal side end in the lengthwise direction of the absorbent body collapses to the non-skin side, i.e. it collapses outward (outward collapse), the surface on the skin side of the absorbent body separates from the dorsal region forming a gap, and urine can potentially leak out from the gap.

It is an object of the present invention to provide an absorbent article having stretchable side flaps, wherein collapse of the dorsal side end toward the non-skin side can be inhibited, and leakage of excreta from the dorsal region can be prevented.

### SOLUTION TO PROBLEM

The present invention provides the absorbent article of independent claim 1. The dependent claims specify preferred but optional features.

The absorbent article of the present invention is as follows. (1) An absorbent article having a lengthwise direction, a widthwise direction and a thickness direction, and including an absorbent body which includes an absorber, and a pair of side flaps which extend outward from both ends in the widthwise direction, on a dorsal side in the lengthwise direction of the absorbent body, wherein each of the pair of side flaps includes: a stretching member which is in the form of a sheet and stretchable in the widthwise direction, a first sheet member which is layered on a surface of a non-skin side of the stretching member, a second sheet member which is layered on a surface of a skin side of the stretching member, and an engaging member which is connected to an outer end in the widthwise direction of the stretching member and extends outward in the widthwise direction, each of the pair of side flaps is joined further toward a skin side than the absorbent body in the thickness direction, and in the lengthwise direction, a dorsal side end of the absorber is situated further toward a dorsal side than an imaginary line connecting together edges on abdominal sides of the engaging members in the widthwise direction.

In the absorbent article, as mentioned above, each of the pair of side flaps are joined further toward the skin side than the absorbent body in the thickness direction, and in the lengthwise direction, the dorsal side end of the absorber is situated further toward the dorsal side than an imaginary line connecting together the edges on the abdominal sides of the engaging members in the widthwise direction.

Since each side flap is joined to the skin side of the absorbent body in the thickness direction, when the engaging member of each of the pair of side flaps is pulled outward in the widthwise direction, the pulling force toward the abdominal side in the lengthwise direction, which is applied to the dorsal side end in the lengthwise direction of the absorbent body, can be more easily directed toward the skin side. In addition, by situating the dorsal side end of the absorber in the lengthwise direction further toward the dorsal side than an imaginary line connecting together the edges on the abdominal sides of the engaging members in the widthwise direction, the absorber acts as a barrier, making force applied to the dorsal side end in the lengthwise direction of the absorbent body less likely to be directed to the non-skin side, and prevents the dorsal side end from bending in a convex manner toward the non-skin side in a buckled state, so that the direction of the applied force tends to be directed to the skin side. This synergistic effect allows the dorsal side end in the lengthwise direction of the absorbent body to collapse to the skin side (inward collapse), thus inhibiting collapse of the dorsal side end in the lengthwise direction toward the non-skin side (outward collapse). Furthermore, since the user no longer encounters a situation where the dorsal side end is collapsed toward the non-skin side, the user may have peace of mind during use without concern regarding leakage of urine from a gap on the dorsal side of the absorbent body.

The absorbent article of the present invention may also be (2) the absorbent article according to (1) above, wherein an intersection angle of crossing between a dorsal side edge in the lengthwise direction of each of the pair of side flaps and an edge in the widthwise direction of the absorbent body is an obtuse angle.

In this absorbent article, the dorsal side edge in the lengthwise direction of each of the pair of side flaps crosses diagonally with the edge in the widthwise direction of the absorbent body. With side flaps having such a construction, when the stretching member has elongated outward in the widthwise direction, the direction of the tensile force that is applied to the edges in the widthwise direction of the absorbent body can be directed in a more diagonal manner, or in other words, it can be directed more toward the abdominal side in the lengthwise direction. By further increasing the component of the tensile force that is directed to the abdominal side in the lengthwise direction, it is possible for the dorsal side end in the lengthwise direction of the absorbent body to be further pulled to the skin side in the thickness direction and to the abdominal side in the lengthwise direction.

The absorbent article of the present invention may also be (3) the absorbent article according to (2) above, wherein the dorsal side edge in the lengthwise direction of each of the pair of side flaps has a near edge portion near an intersection with the edge in the widthwise direction of the absorbent body, and a main edge portion remaining, and a near intersection angle of crossing between the near edge portion and the edge in the widthwise direction of the absorbent body is larger than a main intersection angle of crossing between the main edge portion and the edge in the widthwise direction of the absorbent body .

In this absorbent article, the near edge portion of the dorsal side edge in the lengthwise direction of each of the pair of side flaps crosses with the edge in the widthwise direction of the absorbent body at a more nearly parallel angle compared to the main edge portion. Thus, when the stretching member elongates outward in the widthwise direction, the direction of tensile force in the widthwise direction can be changed from approximately the widthwise direction to approximately the lengthwise direction along the near edge portion. This allows the dorsal side end in the lengthwise direction to be pulled to the abdominal side in the lengthwise direction, on the skin side of the absorbent body.

The absorbent article of the present invention may also be (4) the absorbent article according to any one of (1) to (3) above, further including: a waist stretching member which is in the form of a sheet and stretchable in the widthwise direction, extending in the widthwise direction between one and the other of the pair of side flaps of the absorbent body.

Since this absorbent article has a waist stretching member between one and the other of the pair of side flaps of the absorbent body, then when the pair of side flaps are pulled toward both outer sides in the widthwise direction, the waist stretching member can be caused to be necked inward in the lengthwise direction. This allows the dorsal side end in the lengthwise direction of the absorbent body to more easily collapse toward the abdominal side. The dorsal side end in the lengthwise direction can thus be caused to collapse further toward the skin side.

The absorbent article of the present invention may also be (5) the absorbent article according to any one of (1) to (4) above, wherein the absorbent body includes: a body portion which includes a top sheet, a back sheet and an absorber situated between the top sheet and the back sheet, and a connecting sheet member which connects together the first sheet member or second sheet member of each of the pair of side flaps, wherein in the lengthwise direction, the body portion is joined to the connecting sheet member so that a dorsal side end of the body portion is situated further on an abdominal side than another imaginary line connecting together dorsal side edges of the engaging members of the pair of side flaps in the widthwise direction.

In this absorbent article, it is possible to reduce the rigidity at the portions where pulling force is applied, by not having the top sheet, absorber or back sheet present further to the dorsal side than the other imaginary line connecting together the dorsal side edges in the lengthwise direction of the engaging members in the widthwise direction. This allows the dorsal side end in the lengthwise direction of the absorbent body to more easily collapse toward the abdominal side in the lengthwise direction.

The absorbent article of the present invention may also be (6) the absorbent article according to any one of (1) to (5) above, wherein the dorsal side edge of each of the pair of side flaps in the lengthwise direction are further toward an abdominal side than a dorsal side end of the absorbent body.

In this absorbent article, since the dorsal side edges of each of the pair of side flaps are further toward the abdominal side than the dorsal side end of the absorbent body, the portion of the absorbent body, which is further toward the dorsal side in the lengthwise direction than the other imaginary line connecting together the dorsal side edges of each of the pair of side flaps, can be made longer in the lengthwise direction. As a result, the portion of the absorbent body that folds and collapses toward the skin side can be widened. This allows the dorsal side end in the lengthwise direction of the absorbent body to more easily collapse toward the abdominal side, while leakage of excreta from the dorsal region can be further reduced.

The absorbent article of the present invention may also be (7) the absorbent article according to any one of (1) to (6) above, wherein in each of the pair of side flaps, an elongation rate of the second sheet member is greater than an elongation rate of the first sheet member.

In this absorbent article, when each side flap is pulled to both outer sides in the widthwise direction, since the elongation rate of the second sheet member on the skin side is greater than the elongation rate of the first sheet member on the non-skin side, this allows the first sheet member on the non-skin side with the smaller elongation rate to elongate a relatively larger degree while the second sheet member on the skin side with the larger elongation rate elongates a relatively smaller degree. Each side flap can therefore be caused to curve to the second sheet member side on the skin side. As a result, the dorsal side end of the absorbent body can more easily collapse toward the skin side.

The absorbent article of the present invention may also be (8) the absorbent article according to any one of (1) to (7) above, wherein in each of the pair of side flaps, the second sheet members are mutually connected into an integral whole while the first sheet members are separate without being mutually connected.

In this absorbent article, when the pair of side flaps are respectively pulled to both outer sides in the widthwise direction causing necking of the stretching member, since the second sheet members on the skin side are connected together into an integral whole and the first sheet members are separate without being mutually connected on the non-skin side, it is easier to collapse the portions of the absorbent body on the dorsal side, toward the sides of the connected second sheet members on the skin side. As a result, the dorsal side end of the absorbent body can more easily collapse toward the skin side.

### ADVANTAGEOUS EFFECTS OF INVENTION

According to the present invention, it is possible to provide an absorbent article having stretchable side flaps, wherein collapse of the dorsal side end toward the non-skin side can be inhibited, and leakage of excreta from the dorsal region can be prevented.

### BRIEF DESCRIPTION OF DRAWINGS

FIG. 1 is a plan view showing a construction example of an absorbent article according to an embodiment.
FIG. 2 is a cross-sectional view showing a construction example of an absorbent article according to an embodiment.
FIG. 3 is a cross-sectional view showing a construction example of an absorbent article according to an embodiment.
FIG. 4 is a plan view showing a relationship between one engaging member and the other member in an embodiment.
FIG. 5 is a diagram illustrating a function of a side flap for an embodiment.
FIG. 6 is a diagram illustrating a method of producing an absorbent article according to an embodiment.
FIG. 7 is a diagram illustrating a method of producing an absorbent article according to an embodiment.
FIG. 8 is a diagram illustrating a method of producing an absorbent article according to an embodiment.
FIG. 9 is a diagram illustrating a function of a side flap for an embodiment.
FIG. 10 is a diagram illustrating a function of a side flap for an embodiment.
FIG. 11 is a diagram illustrating a function of a waist stretching member for an embodiment.
FIG. 12 is a cross-sectional view showing another construction example of an absorbent article according to an embodiment.

### DESCRIPTION OF EMBODIMENTS

An absorbent article according to an embodiment will now be explained, using a tape-like (open type) disposable diaper as an example. However, the types and uses of the absorbent article of the present invention are not limited to this example, and the present invention may also be applied to other absorbent articles without departing from the scope of the present invention.

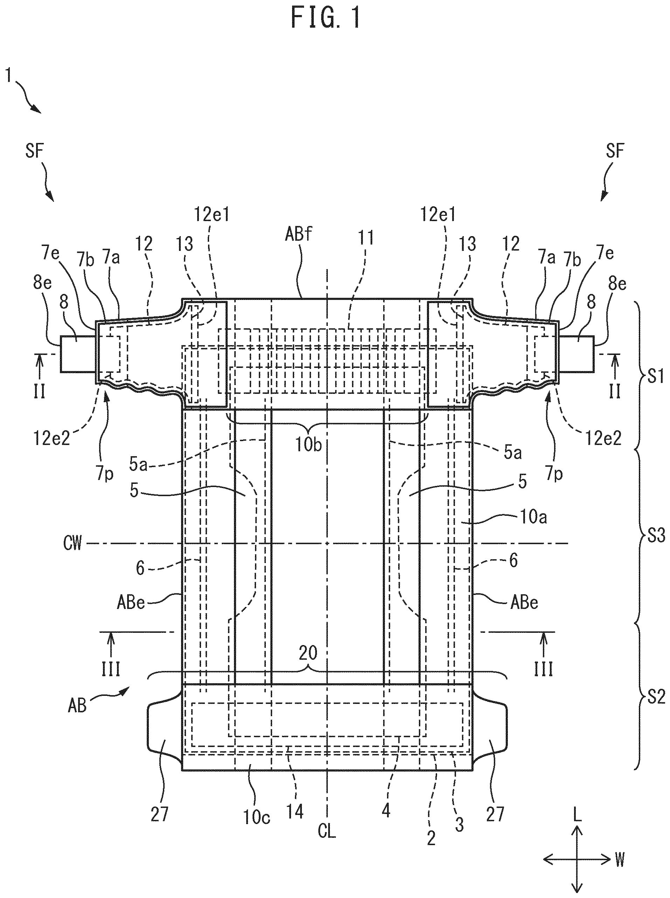

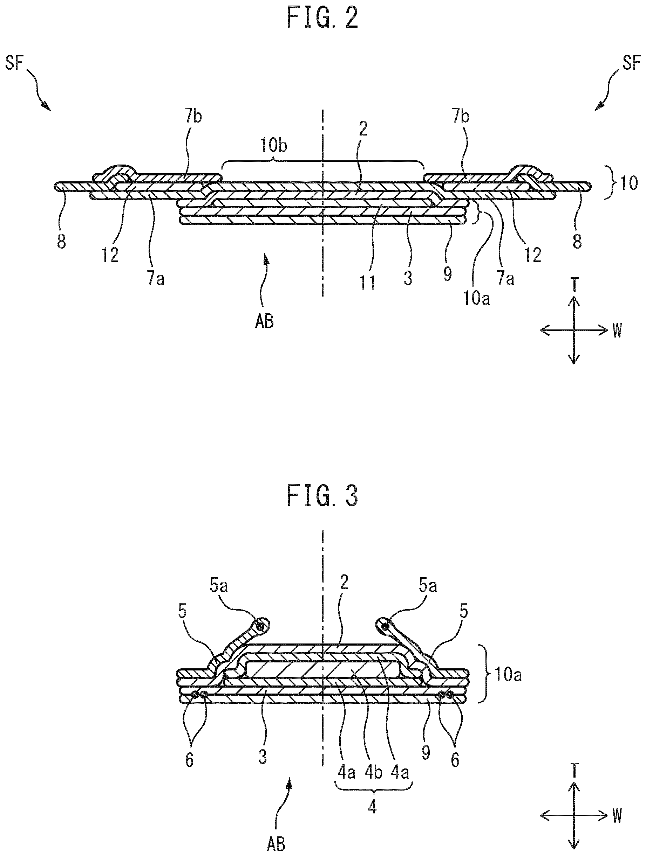

Figs. 1 to 3 are diagrams of an absorbent article 1 (disposable diaper). Fig. 1 is a plan view showing the state when the absorbent article 1 is spread out, Fig. 2 is a cross-sectional view along the line II-II of Fig. 1, and Fig. 3 is a cross-sectional view along the line III-III of Fig. 1. The absorbent article 1 has a lengthwise direction L, a widthwise direction W and a thickness direction T that are mutually perpendicular, and also has a lengthwise center axis line CL running through the center in the widthwise direction W and extending in the lengthwise direction L, and a widthwise center axis line CW running through the center in the lengthwise direction L and extending in the widthwise direction W. Also, the term "plan view" refers to viewing the deployed absorbent article 1 that has been spread out flat, from the upper side in the thickness direction. The terms "skin side" and "non-skin side" refer, respectively, to the side relatively near the side of the wearer's skin and the side relatively away from the side of the wearer's skin, in the thickness direction T of the absorbent article 1 when the absorbent article 1 is worn. The side nearer to and the side further from the lengthwise center axis line CL are the "inner side" and "outer side", respectively, in the widthwise direction W, and the direction toward and the direction away from it are the "inward" direction and the "outward" direction in the widthwise direction W. The side nearer to and the side further from the widthwise center axis line CW are the "inner side" and "outer side", respectively, in the lengthwise direction L, and the direction toward and the direction away from it are the "inward" direction and the "outward" direction in the lengthwise direction L. The term "in-plane direction" is an orientation that includes the lengthwise direction L and widthwise direction W. These definitions also apply to the materials and members that compose the absorbent article 1. Fig. 1 is a diagram of the absorbent article 1 as seen from the skin side.

The absorbent article 1 also has, in the lengthwise direction L, a dorsal side girth region S1 corresponding to the girth around the dorsal side of the wearer, an abdominal side girth region S2 corresponding to the girth around the abdominal side of the wearer, and a crotch region S3 located between the dorsal side girth region S 1 and the abdominal side girth region S2, corresponding to the crotch region of the wearer. The dorsal side girth region S1, crotch region S3 and abdominal side girth region S2 are regions each constituting about 1/3 of the lengthwise direction L. The absorbent article 1 includes an absorbent body AB extending from the dorsal side girth region S 1 to the abdominal side girth region S2 in the lengthwise direction L, a pair of side flaps SF, SF extending outward from both ends in the widthwise direction W of the absorbent body AB, in the dorsal side girth region S1. In the absorbent article 1, a pair of engaging members 8, 8 of the pair of side flaps SF, SF in the dorsal side girth region S1 engage with a target member 14 of the absorbent body AB in the abdominal side girth region S2, for fitting of the diaper. The crotch region S3 may also be narrowed inward in the widthwise direction W. The direction toward the dorsal side girth region S 1 and abdominal side girth region S2 in the lengthwise direction L may also be referred to as the "dorsal side" and "abdominal side", respectively, or may be referred to as the "rear side" and "front side", respectively.

The absorbent body AB of the absorbent article 1 includes a top sheet 2 situated on the skin side, a back sheet 3 situated on the non-skin side, and an absorber 4 situated between the top sheet 2 and the back sheet 3. The top sheet 2 is a liquid-permeable sheet. The top sheet 2 may be any desired liquid-permeable sheet, such as, for example, a liquid-permeable nonwoven fabric or woven fabric, a liquid-permeable pore-formed synthetic resin film, or a composite sheet thereof. The back sheet 3 is a liquid-impermeable sheet. The back sheet 3 may be any desired liquid-impermeable sheet, such as, for example, a liquid-impermeable nonwoven fabric or synthetic resin film, a composite sheet thereof, an SMS nonwoven fabric, or the like. The absorber 4 is a fluid-absorbing and liquid-retaining material, and for this embodiment, it includes an absorber core 4b and absorber core wraps 4a, 4a that subsume the absorber core 4b. The absorber 4 may be pulp fiber, synthetic fiber, an absorbing polymer or the like. The absorber 4 and the top sheet 2 and back sheet 3 are each joined with an adhesive, the top sheet 2 and back sheet 3 being joined by an adhesive at their peripheries. The adhesive used for joining between the top sheet 2, absorber 4 and back sheet 3 may be a known material that is commonly used in absorbent articles 1, such as a thermoplastic adhesive.

The absorbent body AB further includes a pair of anti-leakage walls 5, 5, a pair of leg expandable-and-shrinkable members 6, 6 and an outer sheet 9. The pair of anti-leakage walls 5, 5 cover the surfaces of both ends of the top sheet 2 in the widthwise direction W, while being formed on the inner side portions of the pair of side sheets extending along the lengthwise direction L. Each of the pair of anti-leakage walls 5, 5 has its fixed end at the outer end in the widthwise direction W fixed on the surface of the end in the widthwise direction W of the top sheet 2, and has its free end forming a gather that is stretchable at the inner side end in the widthwise direction W. Near each of the free ends, there are situated linear elastic solids 5a such as rubber, extending along the lengthwise direction L. The outer sheet 9 is disposed on the non-skin side of the back sheet 3 and reinforces the back sheet 3 while improving its hand feel. The pair of leg expandable-and-shrinkable members 6, 6 are linear elastic solids such as rubber extending along the lengthwise direction L on both sides in the widthwise direction W of the absorbent body AB in the crotch region S3, and they are situated between the outer sheet 9 and back sheet 3, for example, causing the portions contacting with the femora of the wearer to be stretched in the lengthwise direction L. A liquid-impermeable sheet is an example for the anti-leakage walls 5, and a hydrophobic, air-permeable sheet is an example for the outer sheet 9.

The absorbent body AB further includes a waist stretching member 11 in the dorsal side girth region S1. The waist stretching member 11 is a member in the form of a sheet such as an elastomer having stretchability in the widthwise direction W, and it is disposed on the inner sides of the pair of side flaps SF, SF in the widthwise direction W and functions as a waist gather. The waist stretching member 11 is attached using an adhesive to any location of the surface on the skin side of the top sheet 2 and the surface on the non-skin side of the back sheet 3, between the top sheet 2 and the back sheet 3 in the dorsal side girth region S1. The waist stretching member 11 is disposed either partially overlapping, or not overlapping, with the absorber 4 in the plan view. For this embodiment, the waist stretching member 11 is disposed between the top sheet 2 and the back sheet 3 in such a manner that the portion of the waist stretching member 11 on the inner side in the lengthwise direction L overlaps with the absorber 4 in the thickness direction T. This can improve the fitting property of the absorbent article 1 in the girth region during wear.

Each of the pair of side flaps SF, SF is joined in the thickness direction T to the surface of the absorbent body AB on the skin side at both ends in the widthwise direction W, whereby it is joined further toward the skin side than the absorbent body AB. The side flap SF includes a stretching member 12 in the form of a sheet that is stretchable in the widthwise direction W, and a first sheet member 7a and second sheet member 7b respectively layered on both sides in the thickness direction T of the stretching member 12. The first sheet member 7a is layered on the surface of the non-skin side of the stretching member 12 while the second sheet member 7b is layered on the surface of the skin side of the stretching member 12. Since each side flap SF has the construction described above for this absorbent article 1, stretchability can be imparted to the side flap SF by the stretching member 12, and holding from both sides through the first and second sheet members 7a, 7b helps to impart strength and rigidity to the side flap SF. That is, the side flaps SF can stretch without producing cracks, to deform along the body of the wearer, thus improving the body compatibility of the absorbent article 1.

For this embodiment, the absorbent body AB includes a body portion 10a including the top sheet 2, the absorber 4 and the back sheet 3, and a connecting sheet member 10b that is joined to the surface on the skin side of the dorsal side girth region S1 of the body portion 10a. The pair of side flaps SF, SF are also mutually connected and integrated by the connecting sheet member 10b. In other words, in the widthwise direction W, the inner side edge of one side flap SF of the pair of side flaps SF, SF and one of the edges of the connecting sheet member 10b are connected, while the inner side edge of the other side flap SF and the other edge of the connecting sheet member 10b are connected. For example, the inner side edge of the first sheet member 7a of one side flap SF and one of the edges of the connecting sheet member 10b are connected, while the inner side edge of the first sheet member 7a of the other side flap SF and the other edge of the connecting sheet member 10b are connected. For this embodiment in particular, the first sheet member 7a of the one side flap SF, the connecting sheet member 10b and the first sheet member 7a of the other side flap SF are integrated, being formed of a single sheet. This is preferred as it facilitates formation of the pair of side flaps SF, SF, and helps to equalize the distances of the pair of side flaps SF, SF from the widthwise center axis line CW in the lengthwise direction L, thereby facilitating production of the absorbent article 1. However, alternatively, the inner side edge of the second sheet member 7b of one side flap SF and one of the edges of the connecting sheet member 10b may be connected while the inner side edge of the second sheet member 7b of the other side flap SF and the other edge of the connecting sheet member 10b are connected. The connecting sheet member 10b may also be absent, in which case the pair of side flaps SF, SF will not be connected.

Known types of sheets may be used for the first and second sheet members 7a, 7b, and nonwoven fabrics are used for this embodiment. The basis weights of the first and second sheet members 7a, 7b are 5 g/m² to 100 g/m², for example. For this embodiment, the first and second sheet members 7a, 7b are each subjected to stretching treatment (for example, gear stretching treatment) before the stretching member 12 is layered and the side flaps SF are formed, so that the structure includes recess sections and raised sections along the lengthwise direction L. The first and second sheet members 7a, 7b can thereby deform in response to stretching of the stretching member 12 in the widthwise direction W, without producing cracks or tears. In each of the first and second sheet members 7a, 7b, the interval between recess sections, i.e. the interval between raised sections, in the widthwise direction W, may be between 0.2 mm and 5 mm, for example. In the thickness direction T, the heights of the top parts of the raised sections, in reference to the bottom sections of the recess sections, are between 0.05 mm and 4 mm, for example. For this embodiment, the first sheet member 7a and the second sheet member 7b have approximately the same shape. The first sheet member 7a, stretching member 12 and second sheet member 7b are layered and joined together, while having both edges in the lengthwise direction L and the outer side edges in the widthwise direction W mutually overlapping, to form the each of the side flaps SF. At each of the side flaps SF, the first sheet member 7a and second sheet member 7b are overlapping in the plan view. However, either the first sheet member 7a or the second sheet member 7b may be larger than the other.

The stretching member 12 may be a sheet of a known elastomer material, for example, a sheet of an elastomer resin such as natural rubber, synthetic rubber or rubber foam. For this embodiment, the stretching member 12 has the approximately the same shape as the first and second sheet members 7a, 7b in the lengthwise direction L. The stretching member 12 also has a shape with its outer side edge 12e2 separated by prescribed distance inward from the outer side edges 7e of the first and second sheet members 7a, 7b, in the widthwise direction W. Therefore, the first and second sheet members 7a, 7b, i.e. the side flap SF, extend further outward than the outer side edge 12e2 in the widthwise direction W of the stretching member 12, and have outward extending sections 7p that do not include the stretching member 12.

For this embodiment, each side flap SF is subjected to stretching treatment (for example, gear stretching treatment), with the first sheet member 7a, stretching member 12 and second sheet member 7b layered. This results in formation of a stretching region 13 having recess sections and raised sections along the lengthwise direction L, on the side flap SF. The first sheet member 7a and second sheet member 7b of the side flap SF are more deformable in the widthwise direction W due to the action of the stretching region 13. At the side flap SF, the first sheet member 7a, stretching member 12 and second sheet member 7b are integral and are more stretchable in the widthwise direction W. However, the stretching region 13 does not overlap with the outward extending sections 7p or with the engaging members 8. Consequently, the bonding sections between the engaging members 8 and the other members are not damaged by stretching treatment. This allows adhesion between the engaging members 8 and the other members to be maintained, and can prevent tearing of the side flap SF at the laminated sections that are subjected to more force when the engaging members 8 have been pulled.

For this embodiment, the inner side edge 12e1 of the stretching member 12 is situated further on the inner side than the edge ABe of the absorbent body AB, in the widthwise direction W. As a result, the distance between the inner side edges of the pair of side flaps SF, SF in the widthwise direction W can be shortened. Thus, when the pair of side flaps SF, SF have been pulled to both outer sides in the widthwise direction W, the tensile force caused by the pair of side flaps SF, SF is more easily transmitted to the absorbent body AB. The fitted feel of the waist portion of the absorbent article 1 can thus be improved, and compatibility of the absorbent article 1 with the body can be improved. For this embodiment, the inner side edges of the first and second sheet members 7a, 7b are also situated further on the inner side than the edge ABe of the absorbent body AB, in the widthwise direction W. As a result, the strength of the inner side ends of each of the pair of side flaps SF, SF in the widthwise direction W can be increased. This allows stable elongation without cracking or tearing at the inner side end of the side flap SF in the widthwise direction W, when it has been attempted to pull the pair of side flaps SF, SF to both outer sides in the widthwise direction W.

For this embodiment, each side flap SF further includes an engaging member 8 as mentioned above. The engaging member 8 is connected to the outer side edge of the stretching member 12 and the outward extending sections 7p in the widthwise direction W, and extends from the outer side edges 7e in the widthwise direction W of the first and second sheet members 7a, 7b. The engaging member 8 is joined with an adhesive, for example, at a location between the first sheet member 7a and the second sheet member 7b in the thickness direction T, on the non-skin side of the first sheet member 7a or on the skin side of the second sheet member 7b. For this embodiment, it is disposed between the first sheet member 7a and the second sheet member 7b, further toward the skin side than the stretching member 12. Examples for the engaging member 8 include synthetic resin or nonwoven fabric sheet members, with hook-and-loop fasteners or adhesive tape being disposed in the region further outward in the widthwise direction W than the outward extending section 7p.

For this embodiment, the absorbent article 1 further includes, in the abdominal side girth region S2, a pair of abdominal side flaps 27, 27 that extend to both outer sides in the widthwise direction W of the body portion 10a of the absorbent body AB, an abdominal side connecting sheet 20 that is joined to the surface on the skin side of the body portion 10a and connects the pair of abdominal side flaps 27, 27 together, and a target member 14 that is disposed on the non-skin side of the body portion 10a. The pair of abdominal side flaps 27, 27 and the target member 14 are sheets where the engaging members 8, 8 are to be connected, the target member 14 being a loop of a hook-and-loop fastener when the engaging members 8, 8 are hooks of hook-and-loop fasteners, for example, while the abdominal side flaps 27 are nonwoven fabrics.

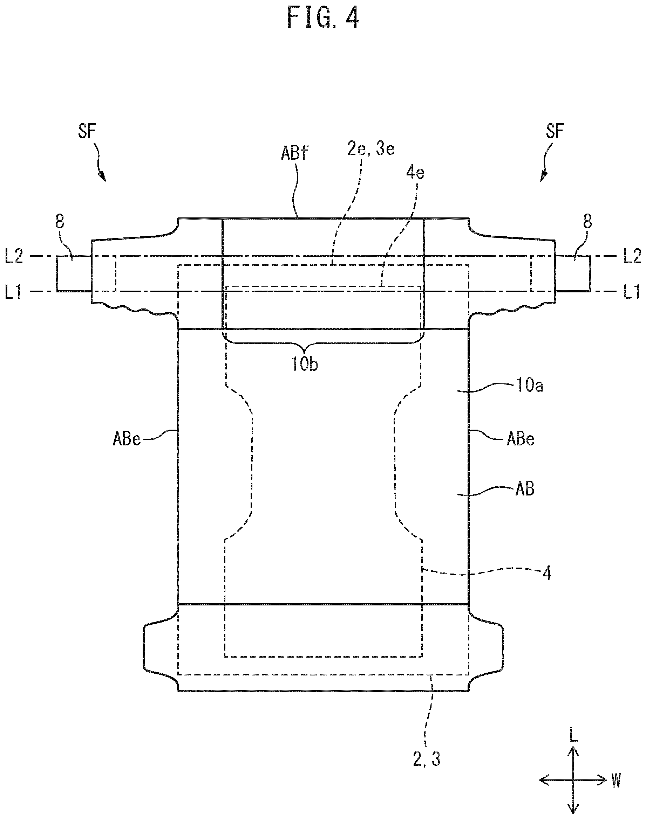

Fig. 4 is a plan view showing the relationship between the engaging members and other members in an embodiment. For this embodiment, an imaginary line L1 is shown as an imaginary line connecting the edges on the abdominal side in the lengthwise direction L of the pair of engaging members 8, 8, together in the widthwise direction W, and an imaginary line L2 is shown as an imaginary line connecting the dorsal side edges together in the widthwise direction W. In this absorbent article 1, the dorsal side end 4e of the absorber 4 in the lengthwise direction L is located further to the dorsal side than the imaginary line L1, and as shown in Fig. 2, each of the pair of side flaps SF, SF is joined further toward the skin side than the absorbent body AB in the thickness direction T. In the absorbent article 1, when the engaging members 8 of the pair of side flaps SF, SF are pulled outward in the widthwise direction W, the end ABf on the outer side (dorsal side) in the lengthwise direction L of the absorbent body AB can be collapsed to the skin side in the thickness direction T (inward collapse). This can inhibit collapse toward the non-skin side (outward collapse) of the end ABf on the outer side of the absorbent body AB in the lengthwise direction L. The reason for this is as follows.

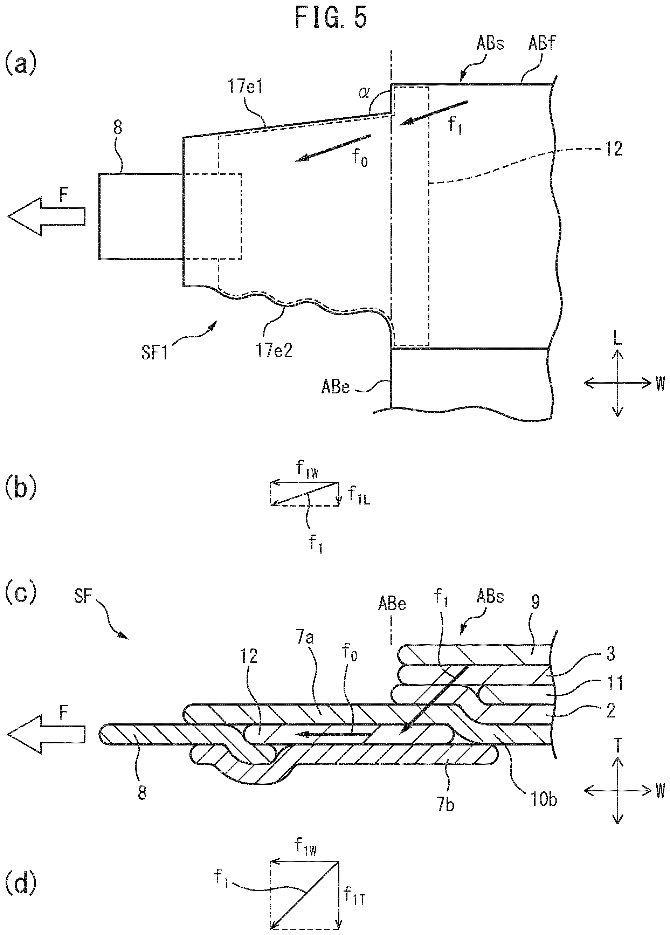

Fig. 5 is a diagram illustrating the function of a side flap SF for an embodiment. Fig. 5(a) is a plan view of the side flap SF, Fig. 5(b) shows components of the force f₁ of Fig. 5(a), Fig. 5(c) is a cross-sectional view of Fig. 5(a), and Fig. 5(d) shows components of the force f₁ of Fig. 5(c). When the engaging members 8 of each of the pair of side flaps SF, SF are pulled outward in the widthwise direction W (arrow F in the drawings), the stretching member 12 layered on the engaging members 8 is pulled in the widthwise direction W. As a result, the portions of the stretching member 12 that are not layered on the engaging members 8 are pulled diagonally with respect to the widthwise direction W, toward the portions that are layered on the engaging members 8, as seen in the plan view. In other words, the portions of the stretching member 12 that are not layered on the engaging members 8 and are on the inner side in the lengthwise direction L are pulled by a certain force toward the portions overlapping with the engaging members 8, and outward in the widthwise direction W and outward in the lengthwise direction L, as seen in the plan view. Meanwhile, the portions of the stretching member 12 that are not layered on the engaging members 8 and that are on the outer side in the lengthwise direction L are pulled by a certain force f₀ toward the engaging members 8, outward in the widthwise direction W and inward in the lengthwise direction L. As a result, an end ABs located on the dorsal side in the lengthwise direction L and the outer side in the widthwise direction W of the absorbent body AB, where the portions of the stretching member 12 pulled by force f₀ are joined, is also pulled by the force f₁ in the same three-dimensional direction as the force f₀. The force f₁ applied to the end ABs has a component f_{1W} in the widthwise direction W and a component f_{1L} in the lengthwise direction L, as well as a component f_{1T} in the thickness direction T. The component f_{1W} is the outward component in the widthwise direction W, the component f_{1L} is the inward component in the lengthwise direction L, and the component f_{1T} is the component oriented to the skin side in the thickness direction T. The component f_{1T} is directed not to the non-skin side but to the skin side in the thickness direction T because the side flaps SF are situated on the skin side of the absorbent body AB in the thickness direction T, as shown in Fig. 5(c). Therefore, when the engaging members 8 of each of the pair of side flaps SF, SF are pulled outward in the widthwise direction W, the force is applied to the end ABs toward the inner side in the lengthwise direction L and toward the skin side in the thickness direction T. Thus, pulling force f₁ toward the abdominal side in the lengthwise direction L can be easily directed to the skin side in the thickness direction T. In addition, by situating the dorsal side end 4e of the absorber 4 in the lengthwise direction L further to the dorsal side than the imaginary line L1, the absorber 4 can act as a barrier on the non-skin side. Since such a barrier makes it difficult for force f₁ applied to the end ABs to be directed to the non-skin side, and can also prevent the area near the end ABf on the dorsal side of the absorbent body AB in the lengthwise direction L from being in a buckled state and bending in a convex manner to the non-skin side in the thickness direction T, the direction of the force f₁ can be even more easily directed to the skin side. These synergistic effects can cause the end ABs to collapse to the skin side in the thickness direction T (inward collapse), thereby allowing the end ABf on the outer side (dorsal side) of the absorbent body AB in the lengthwise direction L to be collapsed to the skin side in the thickness direction T. Thus, collapse toward the non-skin side (outward collapse) of the end ABf on the outer side of the absorbent body AB in the lengthwise direction L can be inhibited. Furthermore, since the user no longer encounters a situation where the end ABf on the dorsal side is collapsed toward the non-skin side, the user may have peace of mind during use without concern regarding leakage of urine from a gap on the dorsal side of the absorbent body AB in the lengthwise direction L.

As a preferred mode of this embodiment, the intersection angle α of crossing between the dorsal side edge 17e1 in the lengthwise direction L of each of the pair of side flaps SF, SF and the edge ABe in the widthwise direction W of the absorbent body AB, is obtuse angles (α > 90°). The intersection angle α is the intersection angle between the edge 17e1 and edge ABe as measured at the joining point that is furthest to the dorsal side in the lengthwise direction L, on the outside boundary in the widthwise direction W, at the joining section between the side flap SF and the absorbent body AB. When the joining point is further on the inner side in the widthwise direction W than the edge ABe of the absorbent body AB, the intersection angle α is the angle of intersection between the edge 17e1 and an imaginary line running through the joining point and parallel to each edge ABe. That is, the edge 17e1 crosses diagonally with the edge ABe. With side flap SF having such a construction, when the stretching member 12 has elongated outward in the widthwise direction W, the direction of the force f₁ (tensile force) that is applied to the end ABs of the absorbent body AB in the widthwise direction W can be directed diagonally, i.e. it can be directed more toward the abdominal side in the lengthwise direction L. As a result, by further increasing the component f_{1L} of the force f₁ (tensile force) that is directed to the abdominal side in the lengthwise direction L, it is possible for the dorsal side end ABf of the absorbent body AB in the lengthwise direction L to be further pulled to the skin side in the thickness direction T and to the abdominal side in the lengthwise direction L.

According to another preferred mode of this embodiment, the body portion 10a is joined to the connecting sheet member 10b in such a manner that the dorsal side end of the body portion 10a of the absorbent body AB, i.e. the dorsal side ends 2e, 4e, 3e of the top sheet 2, absorber 4 and back sheet 3, in the lengthwise direction L, are located further toward the abdominal side than the imaginary line L2. In the absorbent article 1 having such a construction, since the top sheet 2, absorber 4 and back sheet 3 are not present further toward the dorsal side in the lengthwise direction L than the imaginary line L2, it is possible to lower the rigidity of the portions where pulling force f₁ is applied (the ends ABs and ABf). This allows the dorsal side end ABf of the absorbent body AB in the lengthwise direction L to more easily collapse toward the abdominal side in the lengthwise direction L.

According to yet another preferred mode of this embodiment, the dorsal side edge 17e1 of each of the pair of side flaps SF, SF in the lengthwise direction L is further on the abdominal side than the dorsal side end ABf of the absorbent body AB. In the absorbent article 1 having such a construction, the portion of the absorbent body AB that is further toward the dorsal side in the lengthwise direction L than the imaginary line L2 can be lengthened in the lengthwise direction L. As a result, the portion of the absorbent body AB that fold and collapse toward the skin side can be widened. Thus, when the dorsal side end ABf of the absorbent body AB in the lengthwise direction L has collapsed inwardly, the portion on the dorsal side of the wearer can be more widely covered. This allows the dorsal side end ABf of the absorbent body AB in the lengthwise direction L to more easily collapse toward the abdominal side, while leakage of excreta from the dorsal region can be further reduced.

According to yet another preferred mode of this embodiment, at each of the pair of side flaps SF, SF, the elongation rate of the second sheet member 7b on the skin side is greater than the elongation rate of the first sheet member 7a on the non-skin side. In the absorbent article 1 having such a construction, when the pair of side flaps SF, SF are pulled to both outer sides in the widthwise direction W, the first sheet member 7a on the non-skin side with the smaller elongation rate can elongate to a relatively larger degree while the second sheet member 7b on the skin side with the larger elongation rate can elongate to a relatively smaller degree. As a result, each side flap SF can be caused to curve to the second sheet member 7b side on the skin side. This allows the dorsal side end ABf of the absorbent body AB in the lengthwise direction L to even more easily collapse toward the skin side.

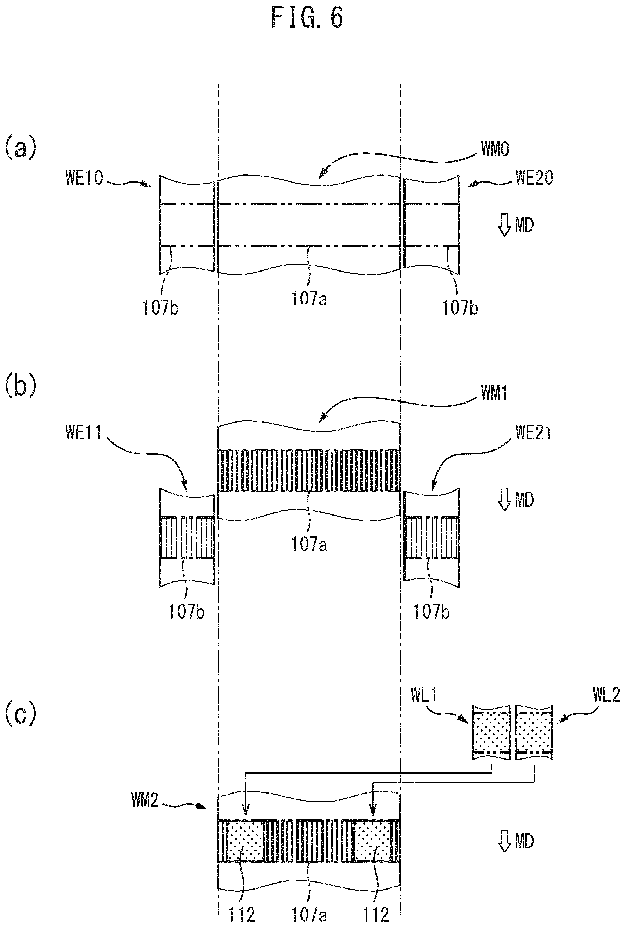

An example of a method of producing an absorbent article according to this embodiment will now be described. Fig. 6 to Fig. 8 schematically show an example of the construction of a sheet or semi-processed web, for explanation of the method of producing an absorbent article. Fig. 6 to Fig. 8 show each sheet or semi-processed web being transported along the machine direction (MD), from top to bottom in the diagrams, showing portions of each sheet or semi-processed web running continuously in the machine direction MD. This production method includes a cutting apart step, a side sheet stretching and joining step, a center sheet stretching step, a stretching member placement step, an engaging member joining step, a folding step, a semi-processed web stretching step, a shaping step and an absorbent body forming step. Incidentally, for this embodiment, the machine direction, the cross-machine direction (CD) perpendicular to the machine direction, and the thickness direction (TD) that is perpendicular to the machine direction and the cross-machine direction during the production process, are equivalent to the lengthwise direction L, widthwise direction W and thickness direction T, respectively, of the absorbent article 1 and its materials. Also, the direction toward and the direction away from an imaginary center axis line running in the machine direction through the center in the cross-machine direction of the transport surface are the inward and outward directions in the cross-machine direction.

First, in the cutting apart step, the material sheet in the form of a continuous sheet (not shown), is supplied to a cutting device (not shown) and is cut apart. Thus, as shown in Fig. 6(a), there are formed a center sheet WM0 and first and second side sheets WE10, WE20, adjacent to the one side and the other side, respectively, in the cross-machine direction of the center sheet WM0. The center sheet WM0 includes a portion 107a that includes the connecting sheet member 10b and the pair of first sheet members 7a, 7a. The first and second side sheets WE10, WE20 each include a portion 107b including a second sheet member 7b. Next, the center sheet WM0 is supplied to the center sheet stretching step, and the first and second side sheets WE10, WE20 are supplied to the side sheet stretching and joining step.

In the subsequent center sheet stretching step, the center sheet WM0 as a continuous sheet is supplied to a stretching apparatus (not shown), and stretching treatment (for example, gear stretching treatment) is carried out in the cross-machine direction. This forms a center sheet WM1 that is stretchable in the cross-machine direction, as shown in Fig. 6(b). The center sheet WM1 includes the portion 107a. The center sheet WM1 is supplied to the stretching member placement step.

In the side sheet stretching and joining step (first stage), the first and second side sheets WE10, WE20 as continuous sheets are supplied to a stretching apparatus (not shown), and stretching treatment (for example, gear stretching treatment) is carried out in the cross-machine direction. This forms first and second side sheets WE11, WE21 that are stretchable in the cross-machine direction, as shown in Fig. 6(b). The first and second side sheets WE11, WE21 include the portion 107b. The first and second side sheets WE11, WE21 are transported to a press roll (not shown). The side sheet stretching and joining step (second stage) will be explained below.

In the center sheet stretching step and the side sheet stretching and joining step (first stage), the shapes of the recess sections and raised sections formed in the sheet by the stretching treatment (the intervals between the raised sections or recess sections, the heights of the top parts of the raised sections compared to the bottom sections of the recess sections, and the angles formed by the directions of extension of the recess sections or raised sections with respect to the machine direction), may be the same or they may be different.

In the subsequent stretching member placement step, a pair of stretching member sheets WL1, WL2 as continuous sheets are supplied to a cutting device (not shown), and are cut to prescribed dimensions in the machine direction. A pair of stretching members 112 are thus formed. The stretching members 112 are essentially the stretching members 12. Also, the surfaces of the pair of stretching members 112 that are coated with an adhesive are pressed against the center sheet WM1 with a press roll, thus attaching them. This causes the pair of stretching members 112 to be situated in a manner overlapping with the center sheet WM1 in the thickness direction, as shown in Fig. 6(c). The center sheet WM2 is thus formed. The center sheet WM2 is supplied to the engaging member joining step.

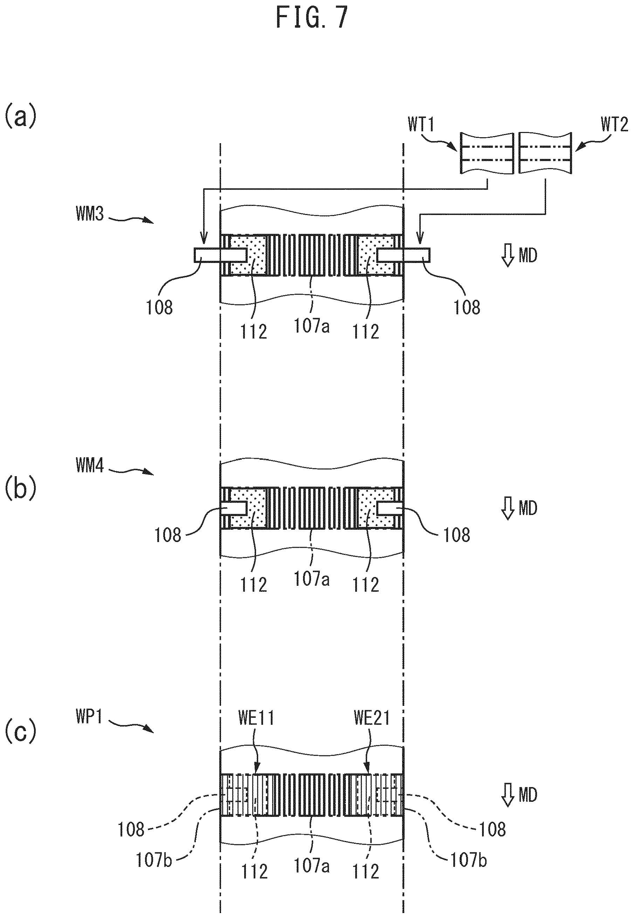

In the subsequent engaging member joining step, a pair of engaging member sheets WT1, WT2 as continuous sheets are supplied to a cutting device (not shown), and are cut to prescribed dimensions in the machine direction. A pair of engaging members 108 are thus formed. The engaging members 108 are essentially the engaging members 8. Also, the surfaces of the pair of engaging members 108 that are coated with an adhesive are pressed against the center sheet WM2 with a press roll, thus attaching them. This causes the pair of engaging members 108 to be situated in a manner overlapping with the pair of stretching members 112 and the center sheet WM2 in the thickness direction TD, as shown in Fig. 7(a). The center sheet WM3 is thus formed. The center sheet WM3 is supplied to the folding step.

In the subsequent folding step, as shown in Fig. 7(b), the extended sections of the pair of engaging members 108, 108 that extend from the locations of both edges of center sheet WM3 in the cross-machine direction toward both outer sides are folded inward by a folding apparatus (not shown) in the cross-machine direction. In this case, each extended section of the engaging member 108 is folded in a manner so as to cover the surface of the center sheet WM3 opposite to the surface on which the stretching members 112 are situated. The center sheet WM4 is thus formed. The center sheet WM4 is supplied to the latter process of the side sheet stretching and joining step.

Next, in the side sheet stretching and joining step (second stage), an adhesive (for example, a hot-melt adhesive) is coated onto one surface of the first and second side sheets WE11, WE21 by a coating applicator (not shown). Press rolls press the surfaces of the first and second side sheets WE11, WE21 that have been coated with the adhesive, onto the surface of the center sheet WM4 on which the stretching members 112 are located. For this embodiment, in the cross-machine direction, the center sheet WM4 and the first side sheet WE11 and second side sheet WE21 are layered in such a manner that the outer side edges of the first side sheet WE11 and the outer side edges of the second side sheet WE21 overlap at both edges of the center sheet WM4. Thus, as shown in Fig. 7(c), a semi-processed sheet WP1 is formed having each of the first and second side sheets WE11, WE21 layered at both ends of the center sheet WM4. The semi-processed sheet WP1 is supplied to the semi-processed web stretching step.

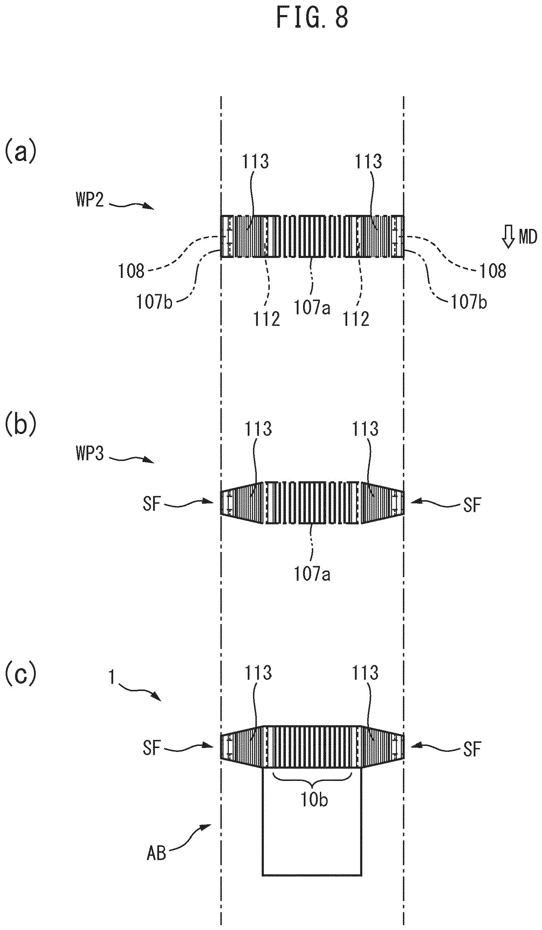

In the subsequent semi-processed web stretching step, the semi-processed sheet WP1 is supplied to a stretching apparatus (not shown). Also, as shown in Fig. 8(a), all or portions of both side sections of the semi-processed sheet WP1 (the locations where the first and second side sheets WE11, WE21 are layered) are subjected to stretching treatment (for example, gear stretching treatment) in the cross-machine direction, by a stretching apparatus. This forms a semi-processed sheet WP2 having stretchable stretching regions 113 formed in the cross-machine direction over all or portions of both ends of the semi-processed sheet WP1. The semi-processed sheet WP2 is supplied to the shaping step.

In the subsequent shaping step, the semi-processed sheet WP2 is supplied to a shaping apparatus (not shown). Also, as shown in Fig. 8(b), both side sections of the semi-processed sheet WP2 (the locations where the first and second side sheets WE11, WE21 are layered) are shaped into approximately trapezoidal shapes by the shaping apparatus. This forms a semi-processed sheet WP3 including a pair of side flaps SF, SF at both ends of the semi-processed sheet WP2.

Next, the semi-processed sheet WP3 is cut off to prescribed dimensions in the machine direction by a cutting device (not shown), and each semi-processed sheet WP3, i.e. each individual separated pair of side flaps SF, SF connected by connecting sheet member 10b are formed. The each individual separated pair of side flaps SF, SF connected by connecting sheet member 10b are transported to the absorbent body forming step.

In the absorbent body forming step, as shown in Fig. 8(c), the pair of side flaps SF, SF connected by the connecting sheet member 10b are joined to the body portion 10a of a separately formed absorbent body AB. However, the pair of side flaps SF, SF connected by the connecting sheet member 10b may be joined to the body portion 10a with the skin side-facing surface and the non-skin side-facing surface reversed. The absorbent article 1 is produced by the process described above. Incidentally, this process is only an example, and the embodiment is not limited to this example.

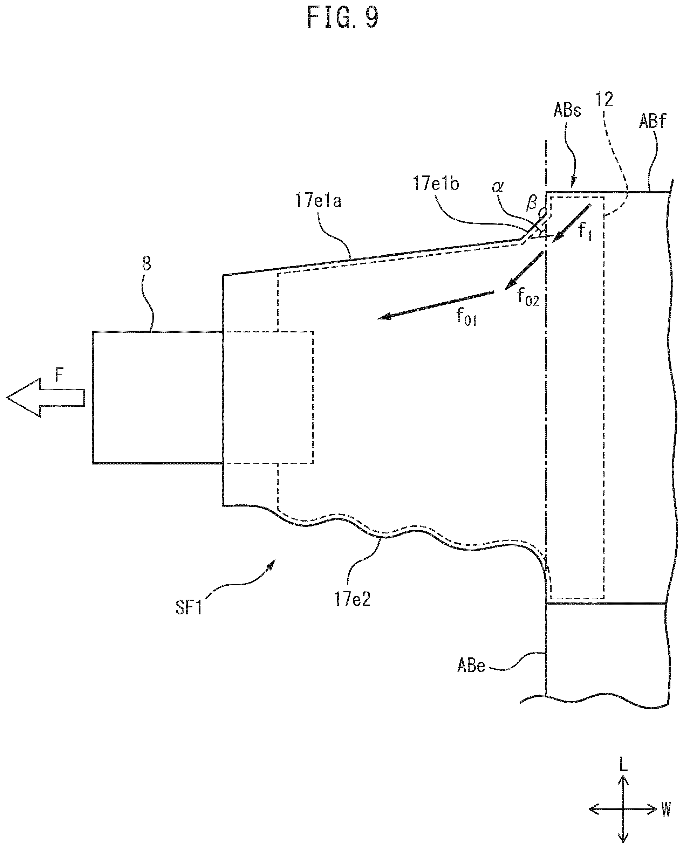

Another preferred mode of this embodiment will now be explained. Fig. 9 is a diagram illustrating the function of a side flap for an embodiment. For this absorbent article 1, the dorsal side edge 17e1 of each of the pair of side flaps SF, SF in the lengthwise direction L has a near edge portion 17elb near the intersection with the edge ABe of the absorbent body AB in the widthwise direction W, and a remaining main edge portion 17ela. The near intersection angle β of crossing between the near edge portion 17elb and the edge ABe of the absorbent body AB in the widthwise direction W is larger than the main intersection angle α of crossing between the main edge portion 17ela (the line extending it inward in the widthwise direction W) and the edge ABe of the absorbent body AB in the widthwise direction W (β > α). However, α > 90°. In other words, the side flap SF of Fig. 9 differs from the side flap SF of Fig. 5 in that it has a near edge portion 17elb.

In this absorbent article 1, the near edge portion 17elb of the dorsal side edge 17e1 of the side flap SF in the lengthwise direction L crosses with the edge ABe of the absorbent body AB in the widthwise direction W at a more nearly parallel angle compared to the main edge portion 17ela (β is nearer to 180 degrees than α). Thus, when the side flap SF, i.e. the stretching member 12, has been pulled outward in the widthwise direction W, the force f₀₁ (tensile force) in the widthwise direction W can change to the force f₀₂ directed along the near edge portion 17elb, or in other words, it can change from the force f₀₁ in approximately the widthwise direction W to the force f₀₂ in approximately the lengthwise direction L. During this time, the end ABs of the absorbent body AB is also pulled by a force f₁ having the same three-dimensional orientation as the force f₀₂. As a result, this can increase the component f_{1L} of the force f₁ applied to the end ABs, which is directed inward in the lengthwise direction L. This allows the dorsal side end ABf in the lengthwise direction L to be more strongly pulled to the abdominal side in the lengthwise direction L, on the skin side of the absorbent body AB, and to be more reliably collapsed on the skin side in the thickness direction T. In the example of Fig. 9, the angle with respect to the edge ABe of the absorbent body AB in the widthwise direction W is changed by dividing the dorsal side edge 17e1 in the lengthwise direction L into two portions at the side flap SF, but the angle with respect to the edge ABe may instead be changed by dividing it into three or more portions.

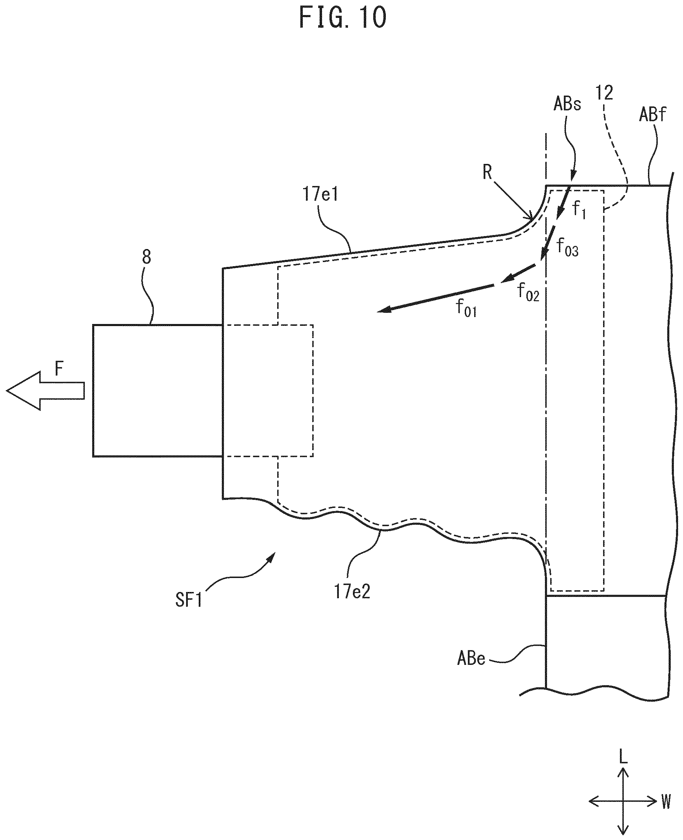

Another preferred mode of this embodiment will now be explained. Fig. 10 is a diagram illustrating the function of a side flap for an embodiment. In this absorbent article 1, the dorsal side edge 17e1 of the side flap SF in the lengthwise direction L has a shape that bends in a convex manner with respect to the edge ABe of the absorbent body AB in the widthwise direction W, near the intersection with the edge ABe of the absorbent body AB in the widthwise direction W. That is, the side flap SF of Fig. 10 differs from the side flap SF of Fig. 5 in that the edge 17e1 has the convex shape R with respect to the edge ABe.

For this absorbent article 1, the portion of the edge 17e1 of the side flap SF that crosses with the edge ABe has a shape that curves in a convex manner with respect to the edge ABe. Thus, when the side flap SF, i.e. the stretching member 12, has been pulled outward in the widthwise direction W, the force f₀₁ (tensile force) in the widthwise direction W can change from the force f₀₁ in approximately the widthwise direction W, via the force f₀₂, to the force f₀₃ in approximately the lengthwise direction L, along the shape of the curve (although in actuality there are multiple continuous stages). During this time, the end ABs of the absorbent body AB is also pulled by a force f₁ having the same three-dimensional orientation as the force f₀₃. As a result this can increase the component f_{1L} of the force f₁ applied to the end ABs, which is directed inward in the lengthwise direction L. This allows the dorsal side end ABf in the lengthwise direction L to be more strongly pulled to the abdominal side in the lengthwise direction L, on the skin side of the absorbent body AB, and to be more reliably collapsed on the skin side in the thickness direction T.

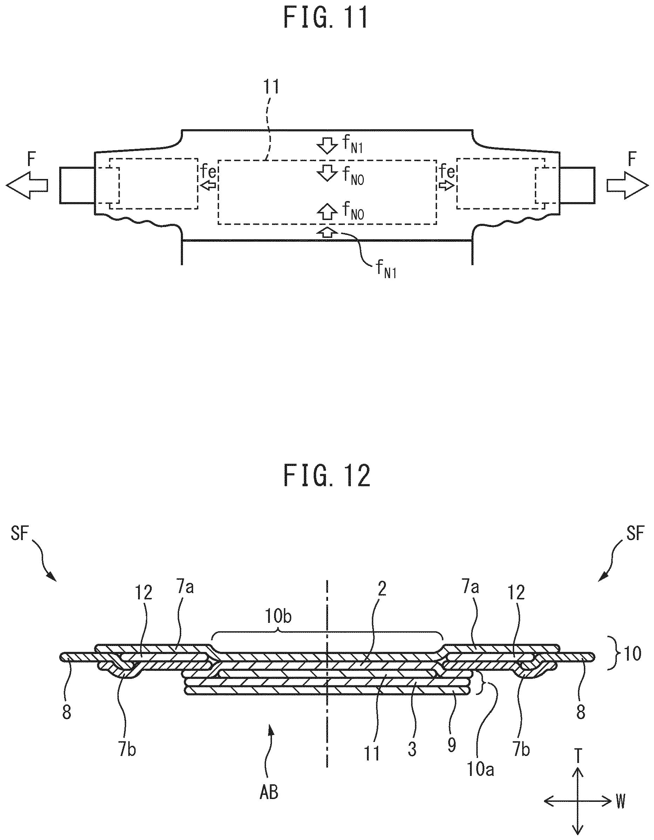

Another preferred mode of this embodiment will now be explained. Fig. 11 is a diagram illustrating the function of a waist stretching member for an embodiment. For this absorbent article 1, the waist stretching member 11 extends in the widthwise direction W, between one and the other of the pair of side flaps SF, SF of the absorbent body AB. For this embodiment, the waist stretching member 11 is disposed so that the outer end of the waist stretching member 11 in the lengthwise direction L is situated further outward in the lengthwise direction L than the imaginary line L1. Thus, when the pair of side flaps SF, SF are pulled in both outer sides in the widthwise direction W (the arrows F in the drawing), the waist stretching member 11 is also pulled to both outer sides in the widthwise direction W (the arrows fe in the drawing). As a result, forces f_{NO}, f_{NO} that narrow the waist stretching member 11 (cause necking in) are produced in the waist stretching member 11 along the lengthwise direction L, causing the waist stretching member 11 to be necked-in in the lengthwise direction L. Thus, the dorsal side end ABf of the absorbent body AB in the lengthwise direction L is pulled further toward the abdominal side in the lengthwise direction L by the force f_{NO}. That is, the dorsal side end ABf of the absorbent body AB in the lengthwise direction L can be made to more easily collapse. The end ABf can thereby be caused to more stably collapse to the skin side. From the viewpoint of increasing the force f_{NO}, f_{NO} that causes narrowing (necking in) of the waist stretching member 11, the waist stretching member 11 is preferably disposed so that the waist stretching member 11 includes the imaginary lines L2, L2 in the lengthwise direction L.

Another preferred mode of this embodiment will now be explained. Fig. 12 is a cross-sectional view showing another construction example of an absorbent article according to an embodiment. For this absorbent article 1, the sheet members 7a on the skin side of the pair of side flaps SF, SF are mutually connected in an integral manner, and the sheet members 7b on the non-skin side are separate without being mutually connected. In this absorbent article 1, when the pair of side flaps are respectively pulled to both outer sides in the widthwise direction causing necking of the stretching member 12, the dorsal side end ABf of the absorbent body AB can more easily collapse to the side of the connected sheet member 7a on the skin side. As a result, the dorsal side end ABf of the absorbent body AB can more easily collapse toward the skin side.

The structure in which the pair of side flaps SF, SF are mutually connected and integrated by the connecting sheet member 10b, i.e. the integrated structure of the side flaps, may join to the body portion 10a in a state that the first sheet member 7a faces the non-skin side, as shown in Fig. 2, or it may join to the body portion 10a in a state that the first sheet member 7a faces the skin side, as shown in Fig. 12. In the absorbent body-forming step of this production method, an absorbent article 1 of this type can be produced by vertically inverting the side on which the structure wherein the pair of side flaps SF, SF and the connecting sheet are integrated is attached to the body portion 10a. In this case, the surface of the skin side of the structure wherein the pair of side flaps SF, SF and connecting sheet are integrated consists of a single first sheet member 7a, and therefore a very skin-soft absorbent article 1 with no catching areas can be produced.

The absorbent article of the present invention is not restricted to the embodiments described above and can incorporate appropriate combinations and modifications without departing from a scope of the present invention.

### REFERENCE SIGNS LIST

1 Absorbent article
7a, 7b Sheet member
8 Engaging member
12 Stretching member
AB Absorbent body
SF Side flap

## Claims

1. An absorbent article (1) having a lengthwise direction (L), a widthwise direction (W) and a thickness direction (T), and comprising an absorbent body (AB) which includes an absorber (4), and a pair of side flaps (SF) which extend outward from both ends in the widthwise direction, on a dorsal side in the lengthwise direction of the absorbent body (AB), wherein
each of the pair of side flaps (SF) comprises:
a stretching member (12) which is in the form of a sheet and stretchable in the widthwise direction,
a first sheet member (7a) which is layered on a surface of a non-skin side of the stretching member (12),
a second sheet member (7b) which is layered on a surface of a skin side of the stretching member (12), and
an engaging member (8) which is connected to an outer end in the widthwise direction of the stretching member (12) and extends outward in the widthwise direction (W),
each of the pair of side flaps (SF) is joined further toward a skin side than the absorbent body (AB) in the thickness direction, and
in the lengthwise direction (L), a dorsal side end (4e) of the absorber (4) is situated further toward a dorsal side than an imaginary line (LI) connecting together edges on abdominal sides of the engaging members (8) in the widthwise direction.

2. The absorbent article (1) according to claim 1, wherein
an intersection angle (α) of crossing between a dorsal side edge (17e1) in the lengthwise direction of each of the pair of side flaps (SF) and an edge (ABe) in the widthwise direction of the absorbent body (AB) is an obtuse angle.

3. The absorbent article (1) according to claim 2, wherein
the dorsal side edge (17e1) in the lengthwise direction of each of the pair of side flaps (SF) has a near edge portion (17elb) near an intersection with the edge (ABe) in the widthwise direction of the absorbent body (AB), and a main edge portion (17ela) remaining, and
a near intersection angle (β) of crossing between the near edge portion (17e1b) and the edge (ABe) in the widthwise direction of the absorbent body (AB) is larger than a main intersection angle (α) of crossing between the main edge portion (17ela) and the edge (ABe) in the widthwise direction of the absorbent body (AB).

4. The absorbent article according to any one of claims 1 to 3, further comprising: a waist stretching member (11) which is in the form of a sheet and stretchable in the widthwise direction (W), extending in the widthwise direction between one and the other of the pair of side flaps (SF) of the absorbent body (AB).

5. The absorbent article (1) according to any one of claims 1 to 4, wherein the absorbent body (AB) comprises:
a body portion (10a) which includes a top sheet (2), a back sheet (3) and an absorber (4) situated between the top sheet (2) and the back sheet (3), and
a connecting sheet member (10b) which connects together the first sheet member (7a) or second sheet member (7b) of each of the pair of side flaps (SF), wherein
in the lengthwise direction (L), the body portion (10a) is joined to the connecting sheet member (10b) so that a dorsal side end of the body portion (10a) is situated further on an abdominal side than another imaginary line (L2) connecting together dorsal side edges of the engaging members (8) of the pair of side flaps (SF) in the widthwise direction.

6. The absorbent article (1) according to any one of claims 1 to 5, wherein
the dorsal side edge (17e1) of each of the pair of side flaps (SF) in the lengthwise direction are further toward an abdominal side than a dorsal side end (ABf) of the absorbent body (AB).

7. The absorbent article (1) according to any one of claims 1 to 6, wherein
in each of the pair of side flaps (SF), an elongation rate of the second sheet (7b) member is greater than an elongation rate of the first sheet member (7a).

8. The absorbent article (1) according to any one of claims 1 to 7, wherein
in the pair of side flaps (SF), the second sheet members (7b) are mutually connected into an integral whole while the first sheet members (7a) are separate without being mutually connected.

## Patentansprüche

1. Absorbierender Artikel (1), der eine Längsrichtung (L), eine Breitenrichtung (W) und eine Dickenrichtung (T) aufweist und Folgendes umfasst: einen Saugkörper (AB), der einen Absorber (4) einschließt, und ein Paar von Seitenklappen (SF), die sich nach außen von beiden Enden in der Breitenrichtung erstrecken, auf einer dorsalen Seite in der Längsrichtung des Saugkörpers (AB), wobei
jede des Paars von Seitenklappen (SF) Folgendes umfasst:
ein Dehnelement (12), das in Form einer Lage vorliegt und in der Breitenrichtung dehnbar ist,
ein erstes Lagenelement (7a), das auf einer Oberfläche einer Nicht-Hautseite des Dehnelements (12) geschichtet ist,
ein zweites Lagenelement (7b), das auf einer Oberfläche einer Hautseite des Dehnelements (12) geschichtet ist, und
ein eingreifendes Element (8), das mit einem äußeren Ende in der Breitenrichtung des Dehnelements (12) verbunden ist und sich nach außen in der Breitenrichtung (W) erstreckt,
jede des Paars von Seitenklappen (SF) weiter in Richtung einer Hautseite als der Saugkörper (AB) in der Dickenrichtung verbunden ist und
in der Längsrichtung (L) sich ein dorsales Seitenende (4e) des Absorbers (4) weiter in Richtung einer dorsalen Seite befindet als eine imaginäre Linie (LI), die Ränder auf abdominalen Seiten der eingreifenden Elemente (8) in der Breitenrichtung miteinander verbindet.

2. Absorbierender Artikel (1) nach Anspruch 1, wobei
ein Schnittwinkel (a) der Kreuzung zwischen einem dorsalen Seitenrand (17e1) in der Längsrichtung von jeder des Paars von Seitenklappen (SF) und einem Rand (ABe) in der Breitenrichtung des Saugkörpers (AB) ein stumpfer Winkel ist.

3. Absorbierender Artikel (1) nach Anspruch 2, wobei
der dorsale Seitenrand (17e1) in der Längsrichtung von jeder des Paars von Seitenklappen (SF) einen randnahen Teil (17e1b) nahe eines Schnittpunkts mit dem Rand (ABe) in der Breitenrichtung des Saugkörpers (AB) und einen restlichen Hauptrandteil (17ela) aufweist und
ein naher Schnittwinkel (β) der Kreuzung zwischen dem randnahen Teil (17e1b) und dem Rand (ABe) in der Breitenrichtung des Saugkörpers (AB) größer ist als ein Hauptschnittwinkel (a) der Kreuzung zwischen dem Hauptrandteil (17e1a) und dem Rand (ABe) in der Breitenrichtung des Saugkörpers (AB).

4. Absorbierender Artikel nach einem der Ansprüche 1 bis 3, der weiter Folgendes umfasst: ein Taillendehnelement (11), das in Form einer Lage vorliegt und in der Breitenrichtung (W) dehnbar ist, das sich in der Breitenrichtung zwischen der einen und der anderen des Paars von Seitenklappen (SF) des Saugkörpers (AB) erstreckt.

5. Absorbierender Artikel (1) nach einem der Ansprüche 1 bis 4, wobei der Saugkörper (AB) Folgendes umfasst:
einen Körperteil (10a), der Folgendes einschließt: eine obere Lage (2), eine hintere Lage (3) und einen Absorber (4), der sich zwischen der oberen Lage (2) und der hinteren Lage (3) befindet, und
ein verbindendes Lagenelement (10b), das das erste Lagenelement (7a) oder zweite Lagenelement (7b) von jeder des Paars von Seitenklappen (SF) miteinander verbindet, wobei
in der Längsrichtung (L) der Körperteil (10a) mit dem verbindenden Lagenelement (10b) derart verbunden ist, dass sich ein dorsales Seitenende des Körperteils (10a) weiter auf einer abdominalen Seite befindet als eine weitere imaginäre Linie (L2), die dorsale Seitenränder der eingreifenden Elemente (8) des Paars von Seitenklappen (SF) in der Breitenrichtung miteinander verbindet.

6. Absorbierender Artikel (1) nach einem der Ansprüche 1 bis 5, wobei
der dorsale Seitenrand (17e1) von jeder des Paars von Seitenklappen (SF) in der Längsrichtung weiter in Richtung einer abdominalen Seite vorliegt als ein dorsales Seitenende (ABf) des Saugkörpers (AB).

7. Absorbierender Artikel (1) nach einem der Ansprüche 1 bis 6, wobei
in jeder des Paars von Seitenklappen (SF) eine Dehnrate des zweiten Lagenelements (7b) größer ist als eine Dehnrate des ersten Lagenelements (7a).

8. Absorbierender Artikel (1) nach einem der Ansprüche 1 bis 7, wobei
in dem Paar von Seitenklappen (SF) die zweiten Lagenelemente (7b) zu einem integralen Ganzen gegenseitig verbunden sind, während die ersten Lagenelemente (7a), ohne gegenseitige Verbindung, getrennt sind.

## Revendications

1. Article absorbant (1) ayant une direction allant dans le sens de la longueur (L), une direction allant dans le sens de la largeur (W) et une direction allant dans le sens de l'épaisseur (T), et comportant un corps absorbant (AB) qui comprend un élément absorbant (4), et une paire de rabats latéraux (SF) qui s'étendent vers l'extérieur depuis les deux extrémités dans la direction allant dans le sens de la largeur, sur un côté dorsal dans la direction allant dans le sens de la longueur du corps absorbant (AB), dans lequel
chacun de la paire de rabats latéraux (SF) comporte :
un élément d'étirement (12) qui est sous la forme d'une feuille et en mesure de s'étirer dans la direction allant dans le sens de la largeur,
un premier élément formant feuille (7a) qui est mis en couche sur une surface d'un côté non orienté vers la peau de l'élément d'étirement (12),
un deuxième élément formant feuille (7b) qui est mis en couche sur une surface d'un côté orienté vers la peau de l'élément d'étirement (12), et
un élément de mise en prise (8) qui est raccordé à une extrémité extérieure dans la direction allant dans le sens de la largeur de l'élément d'étirement (12) et qui s'étend vers l'extérieur dans la direction allant dans le sens de la largeur (W),
chacun de la paire de rabats latéraux (SF) est joint plus encore vers un côté orienté vers la peau par rapport au corps absorbant (AB) dans la direction allant dans le sens de l'épaisseur, et
dans la direction allant dans le sens de la longueur (L), une extrémité côté dorsal (4e) de l'élément absorbant (4) est située plus encore vers un côté dorsal par rapport à une ligne imaginaire (L1) raccordant ensemble des bords sur des côtés abdominaux des éléments de mise en prise (8) dans la direction allant dans le sens de la largeur.

2. Article absorbant (1) selon la revendication 1, dans lequel
un angle d'intersection (a) de croisement entre un bord côté dorsal (17e1) dans la direction allant dans le sens de la longueur de chacun de la paire de rabats latéraux (SF) et un bord (ABe) dans la direction allant dans le sens de la largeur du corps absorbant (AB) est un angle obtus.

3. Article absorbant (1) selon la revendication 2, dans lequel
le bord côté dorsal (17e1) dans la direction allant dans le sens de la longueur de chacun de la paire de rabats latéraux (SF) a une partie bord proche (17elb) près d'une intersection avec le bord (ABe) dans la direction allant dans le sens de la largeur du corps absorbant (AB), et une partie bord principal (17ela) restante, et
un angle d'intersection proche (β) de croisement entre la partie bord proche (17e1b) et le bord (ABe), dans la direction allant dans le sens de la largeur du corps absorbant (AB) est supérieur par rapport à un angle d'intersection principal (a) de croisement entre la partie bord principal (17e1a) et le bord (ABe) dans la direction allant dans le sens de la largeur du corps absorbant (AB).

4. Article absorbant selon l'une quelconque des revendications 1 à 3, comportant par ailleurs : un élément d'étirement au niveau de la taille (11) qui est sous la forme d'une feuille et en mesure de s'étirer dans la direction allant dans le sens de la largeur (W), s'étendant dans la direction allant dans le sens de la largeur entre l'un et l'autre de la paire de rabats latéraux (SF) du corps absorbant (AB).

5. Article absorbant (1) selon l'une quelconque des revendications 1 à 4, dans lequel le corps absorbant (AB) comporte :
une partie formant corps (10a) qui comprend une feuille de dessus (2), une feuille de support (3) et un élément absorbant (4) situé entre la feuille de dessus (2) et la feuille de support (3), et
un élément formant feuille de raccordement (10b) qui raccorde ensemble le premier élément formant feuille (7a) ou le deuxième élément formant feuille (7b) de chacun de la paire de rabats latéraux (SF), dans lequel
dans la direction allant dans le sens de la longueur (L), la partie formant corps (10a) est jointe à l'élément formant feuille de raccordement (10b) de telle sorte qu'une extrémité côté dorsal de la partie formant corps (10a) est située plus encore sur un côté abdominal par rapport à une autre ligne imaginaire (L2) raccordant ensemble des bords côté dorsal des éléments de mise en prise (8) de la paire de rabats latéraux (SF) dans la direction allant dans le sens de la largeur.

6. Article absorbant (1) selon l'une quelconque des revendications 1 à 5, dans lequel
les bords côté dorsal (17e1) de chacun de la paire de rabats latéraux (SF) dans la direction allant dans le sens de la longueur sont plus encore vers un côté abdominal par rapport à une extrémité côté dorsal (ABf) du corps absorbant (AB).

7. Article absorbant (1) selon l'une quelconque des revendications 1 à 6, dans lequel
dans chacun de la paire de rabats latéraux (SF), un taux d'allongement du deuxième élément formant feuille (7b) est supérieur par rapport à un taux d'allongement du premier élément formant feuille (7a).

8. Article absorbant (1) selon l'une quelconque des revendications 1 à 7, dans lequel
dans la paire de rabats latéraux (SF), les deuxièmes éléments formant feuille (7b) sont mutuellement raccordés en un seul tenant alors que les premiers éléments formant feuille (7a) sont séparés sans être mutuellement raccordés.
